# EUROPEAN PATENT APPLICATION

(11) **EP 4 053 090 A1**
(43) Date of publication of application: **07.09.2022**
(21) Application number: 21305261.6
(22) Date of filing: 04.03.2021
(51) Int. Cl.: C04B 28/18, C04B 14/28, C04B 28/04

(54) **COMPOSITE MATERIAL HAVING REINFORCING PROPERTIES**

(71) Applicant: Septodont ou Septodont SAS ou Specialites Septodont, 94100 Saint Maur des Fossés (FR)
(72) Inventor: DJOUDI, Mounir, 35740 Pacé (FR); RICHARD, Gilles, 91560 Crosne (FR); ARTAUD, Laurent, 94340 Joinville Le Pont (FR)
(74) Representative: Icosa

(57) **Abstract**

The present invention relates to the field of hydraulic cements and the hardened materials obtained after hydration of said hydraulic cements. Especially, the present invention concerns a composite material, preferably a hardened composite material, comprising:
- a solid dispersant phase comprising or consisting of calcium silicate hydrates (CSH);
- calcium silicate particles dispersed in the solid dispersant phase;
- a porous intergranular area located between said calcium silicate particles; said porous intergranular area comprising insoluble calcium carbonate particles having a dso granulometry ranging from 1 nm to 1500 nm; preferably from 1 nm to 1000 nm.

The present invention also refers to the method for manufacturing said composite material thereof, and its uses in the medical field or in the non-therapeutical cement field.

## Description

### FIELD OF INVENTION

The present invention relates to the field of hydraulic cements and the hardened materials obtained after hydration of said hydraulic cements. Especially, the present invention deals with a composite material, preferably a hardened composite material, comprising calcium silicate particles dispersed in a solid dispersant phase including calcium silicate hydrates (CSH) and a porous intergranular area located between said calcium silicates particles; said porous intergranular area comprising insoluble calcium carbonate particles with a dso granulometry less than 1,5 µm. The present invention also refers to the method for manufacturing said composite material, and its uses in the medical field or in the non-therapeutical cement field.

### BACKGROUND OF INVENTION

High performance cementitious materials prepared from hydraulic calcium silicate cement such as Portland cement are increasingly finding their use both in medical and non-therapeutical fields (such as construction), worldwide.

Whatever the fields, there is always a need for providing hardened cementitious materials featuring reinforced mechanical properties such as for example higher compressive strength. Especially, there is always a need for providing processes for manufacturing said materials, which are less time consuming and therefore, more economical.

Surprisingly, the Applicant has evidenced that a method for manufacturing a hardened cementitious material comprising at least one mixing step by vibration of a powder phase comprising calcium silicate particles and calcium carbonate particles, with an aqueous phase, enables providing a composite material with reinforcing properties.

Without wishing to be bound by any theory, the Applicant submits that the mixing step by vibration in the manufacturing process of a hardened cementitious material, provides a self-grinding of some of particles contained in the hydraulic cement so that the final composite material owns one or more intergranular areas with submicrometric calcium carbonate particles. The Applicant submits that the presence of these intergranular areas in the final composite material reinforces its mechanical properties.

### SUMMARY

Thus, a first object of the present invention refers to a composite material comprising:
- a solid dispersant phase comprising or consisting of calcium silicate hydrates (CSH);
- calcium silicate particles dispersed in the solid dispersant phase; and
- a porous intergranular area located between said calcium silicate particles; said porous intergranular area comprising insoluble calcium carbonate particles having a dso granulometry ranging from 1 nm to 1500 nm; preferably from 1 nm to 1000 nm.

According to one embodiment, the calcium silicate particles are selected from tricalcium silicate (C3S), dicalcium silicate (C2S) and any combinations thereof; preferably the calcium silicate particles are tricalcium silicate particles.

According to one embodiment, the calcium silicate hydrates are compounds of formula (I):

mCaO.nSiO₂.pH₂O

in which n and m independently range from 1 to 3 and p ranges from 3 to 6; preferably m equals 3, n equals 2 and p equals 3.

According to one embodiment, the composite material further comprises at least one additive, preferably selected from set accelerators, radiopacifiers, pigments, pH stabilizing agents, fillers, texturing/thickening agents, water-reducing agents and mixtures thereof.

According to one embodiment, the radiopacifier is selected from zirconium oxide, bismuth oxide, cerium oxide, barium sulphate, calcium tungstate, titanate dioxide, ytterbium oxide and mixtures thereof; preferably the radiopacifier is zirconium oxide.

According to one embodiment, the porous intergranular area further comprises calcium hydroxide (Ca(OH)₂).

According to one embodiment, the diameter of the pores of the porous intergranular area ranges from more than 0 nm to 50 nm.

According to one embodiment, the dso granulometry of the calcium silicate particles ranges from 1 µm to 10 µm; preferably from 1 µm to 8 µm.

According to one embodiment, the amount of calcium silicate particles ranges from 5 % wt. to 65 % wt. by the total weight of said composite material.

According to one embodiment, the porous intergranular area further comprises calcium silicate hydrates (CSH); preferably porous calcium silicate hydrates (p-CSH).

Another object of the present invention refers to a method for manufacturing a composite material as defined above, comprising at least one mixing step by vibration of a powder phase comprising calcium silicate particles and calcium carbonate particles, with an aqueous phase; in a mass ratio of the powder phase to the aqueous phase ranging from 2 to 4.5.

According to one embodiment, the mixing step is implemented with a vibration frequency ranging from 1 rpm to 15 000 rpm; preferably ranging from 1 rpm to 10 000 rpm, more preferably ranging from 1000 rpm to 6 000 rpm; even more preferably ranging from 3 000 rpm to 5 000 rpm. According to another preferred embodiment, the mixing step is implemented with a vibration frequency ranging from 1 rpm to 15 000 rpm, preferably from 5 000 rpm to 15 000 rpm, more preferably from 10 000 rpm to 15 000 rpm.

According to one embodiment, the mixing step by vibration is implemented during a vibration time ranging from 1 s to 3600 s; preferably from 1 s to 60 s; more preferably during 30 s.

Another object of the present invention refers to the use of a composite material as defined above, as reinforcing material in the non-therapeutical cement field.

Another object of the present invention refers to the composite material as defined above, for use in the medical field, preferably in the dental or the orthopedic field, as a restorative and/or filling material.

### DEFINITIONS

In the present invention, the following terms have the following meanings:
- **"Additive"** refers to any substance added, preferably in a low amount, in a composition for improving its physicochemical properties depending on its use. The additive may for example be selected from radiopacifiers (such as zirconium oxide), setting accelerators (such as calcium oxide, calcium carbonate, calcium chloride), pigments (such as iron oxides), water reducing agents (such as modified polycarboxylates), texturing agents, pH stabilizing agents, surfactants, fillers, and mixtures thereof.
- **"Aqueous"** refers to any compound or composition comprising water and/or moisture.
- **"Calcium silicate"** refers to compounds that can be produced by reacting calcium oxide and silica in various ratios. According to one embodiment, the expression "calcium silicate" refers to compounds made of calcium and silicate, preferably selected from tricalcium silicate, dicalcium silicate or any mixtures thereof; more preferably tricalcium silicate C3S (of formula Ca₃SiO₅), dicalcium silicate C2S (of formula Ca₂SiO₄), or any mixtures thereof.
- **"Calcium silicate particle":** refers to an assembly comprising one or more calcium silicate compounds. The terms "calcium silicate particle" also include assemblies consisting of one or more calcium silicate compounds.
- **"Calcium silicate hydrates"** refers to any products resulting from the hydration of calcium silicate. According to one embodiment, the terms "calcium silicate hydrates" mean a compound of formula (I):

   mCaO.nSiO₂.pH₂O

   in which n and m independently range from 1 to 3, and p ranges from 3 to 6. According to one embodiment, the terms "calcium silicate hydrates" refer to the products resulting from the hydration of dicalcium silicate and/or tricalcium silicate. According to one embodiment, the terms "calcium silicate hydrates" refer to a compound of formula (I) as defined above in which m equals 3, n equals 2 and p equals 3. According to one embodiment, the terms "calcium silicate hydrates" also include calcium hydroxide (Ca(OH)₂). In the present invention, the expression **"porous calcium silicate hydrates (p-CSH)"** means that the calcium silicate hydrates form a matrix with pores; preferably with a pore size ranging from more than 0 nm to less than 1 µm. According to one embodiment, the porous calcium silicate hydrates is a CSH matrix with pores having a pore size higher than 50 nm, preferably ranging from 51 nm to 1 µm; preferably ranging from 51 nm to 100 nm. According to one embodiment, the porous calcium silicate hydrates is a CSH matrix with pores having a pore size ranging from higher than 2 nm to 50 nm. According to one embodiment, the porous calcium silicate hydrates is a CSH matrix with pores having a pore size ranging from more than 0 nm to 2 nm. In the present invention, the expression **"dense calcium silicate hydrates (d-CSH)"** means that the calcium silicate hydrates form a matrix less porous than the porous calcium silicate hydrates as defined above. According to one embodiment, the expression **"dense calcium silicate hydrates (d-CSH)"** means that the calcium silicate hydrates form a matrix without any pores.
- **"Composite material"** refers to any material comprising or consisting of the association of at least two non-miscible components for which the resulting physical and/or chemical properties are different from those of these components taken individually.
- **"Dental cement"** refers to any composition suitable for restorative dentistry that acts as an adhesive to hold together the casting to the tooth structure.
- **"d10 granulometry":** means that 10% of the particles have a mean diameter less than said value. According to one embodiment, the dio size is measured by laser diffraction.
- **"d50 granulometry":** means that 50% of the particles have a mean diameter less than said value. According to one embodiment, the dso size is measured by laser diffraction.
- **"d90 granulometry":** means that 90% of the particles have a mean diameter less than said value. According to one embodiment, the d₉₀ size is measured by laser diffraction.
- **"Dispersant phase":** refers to a chemical medium in which are dispersed particles.
- **"Hardened dental material":** refers to a material suitable for dental applications that is under a solid form. A "hardened dental filling material" especially refers to a hardened dental material suitable for filling dental restoration.
- **"Hydraulic cement":** refers to a cement able to self-harden when contacted with water. According to one embodiment, the cement is a dental hydraulic cement. According to one embodiment, the cement is a non-therapeutical hydraulic cement.
- **"Laser diffraction analysis":** refers to a technique using diffraction patterns of a laser beam passed through a particulate object for determining its size.
- **"Pigment":** refers to any coloring chemical compound that may be natural or synthetic, mineral or organic.
- **"Porous":** refers to a mineral compound comprising mesopores, micropores and/or macropores. The pores of the porous material typically have a pore size ranging from more than 0 nm to 1 µm; for instance from 2 nm to 1 µm.
- **"Portland cement":** refers to a hydraulic material comprising at least two-thirds by mass of calcium silicates, (3 CaO·SiO₂, and 2 CaO·SiO₂) as main component, and comprising additional compounds including aluminum- and/or iron-containing clinker phases (for example, tricalcium aluminate and tetracalcium aluminoferrite).

The expression "Portland cement" includes all the Portland cement compositions well-known by the skilled artisan such as those defined by the European EN 197 norm, and by the International ASTM C150 norm.
- **"Pozzolanic material"** refers, according to ASTM C125, to a natural or artificial siliceous or siliceous and aluminous material which, in itself, possesses little or no cementitious value but which will, in finely divided form and in the presence of water, reacts chemically with calcium hydroxide at ordinary temperature to form compounds possessing cementitious properties. An artificial pozzolanic material may comprise or consisting of, for instance, an industrial by-product such as fly ash, silica fume from silicon smelting, highly reactive metakaolin, slag, burned organic matter residues rich in silica such as rice husk ash, calcined clay, or any mixture thereof. A natural pozzolanic material may comprise or consist of, for instance, volcanic ashes, pumices, zeolites, diatomaceous earths and any mixture thereof. According to one embodiment, the at least one pozzolanic material is selected from the group consisting of fly ash, silica fume, metakaolin, slag, and rice husk ash.
- **"Radiopacifying agent"** or **"radiopacifier":** refers to a substance added to a material in order to make it opaque, especially to make it visible under X-ray imaging.
- **"Reinforcing properties":** refers to any compound able to improve physical properties of a material, preferably to enhance the mechanical properties of a material such as the compressive strength for example.
- **"Silica fume":** refers to an amorphous (non-crystalline) polymorph of silicon dioxide, silica. Silica fume is an ultrafine powder collected as a by-product of the silicon and ferrosilicon alloy production and consists of spherical particles with an average particle diameter of 150 nm. Silica fume is also known as microsilica and its CAS number is 69012-64-2, its EINECS number is 273-761-1.
- **"Water-reducing agent":** refers to a substance able to improve the rheological properties of a composition. Especially, the "water-reducing agent" may be a plastifying or fluidifying agent.

Further, in the present invention, when referring to a range, the following is meant: "ranging from X to Y" means that X and Y are included in the range; "ranging from more than X, up to Y" means that X is not included in the range while Y is included in the range; and "less than X" means that the range includes X or lower values.

### DETAILED DESCRIPTION

### Composite material

This invention relates to a composite material, preferably a hardened composite material. According to one embodiment, the composite material is a hardened cementitious material. According to one embodiment, the composite material is a solid material. According to one embodiment, the composite material of the invention results from the hydration a hydraulic cement. According to one embodiment, the composite material of the invention results from the hydration of a hydraulic cement comprising a calcium silicate compound; preferably selected from tricalcium silicate, dicalcium silicate, Portland cement, mineral trioxide aggregate (MTA) and any combinations thereof; more preferably the composite material of the invention results from the hydration a hydraulic cement comprising tricalcium silicate. According to one embodiment, the composite material of the invention results from the hydration a hydraulic cement comprising a calcium silicate compound as defined above, under the form of particles.

According to one embodiment, the composite material of the invention results from the hydration of a hydraulic cement comprising or consisting of a calcium silicate compound, calcium carbonate and a pozzolanic material, preferably selected from the group consisting of fly ash, silica fume, metakaolin, slag, and rice husk ash.; more preferably is silica fume.

According to one embodiment, the hydration of a hydraulic cement leads to a hardened material. According to one embodiment, the composite material of the invention is a hardened composite material, preferably obtained by the hydration of a hydraulic cement; more preferably obtained by the hydration of a hydraulic cement comprising a calcium silicate compound as defined above.

According to one embodiment, the (hardened) composite material comprises or consists of a dispersant phase, preferably a solid dispersant phase, and calcium silicate particles dispersed in said dispersant phase. According to one embodiment, the (hardened) composite material comprises or consists of a solid dispersant phase and insoluble calcium silicate particles dispersed in said solid dispersant phase. According to one embodiment, the (hardened) composite material comprises or consists of a solid dispersant phase and non-hydrated calcium silicate particles dispersed in said solid dispersant phase. In the present invention, the expression "non-hydrated calcium silicate particles" means that the calcium silicate particles did not react with water or moisture.

According to one embodiment, the (hardened) composite material further comprises at least one additive; preferably selected from setting accelerator, radiopacifiers, pigments, pH stabilizing agents, fillers, texturing/thickening agents, water-reducing agents and mixtures thereof. According to one embodiment, the filler is a pozzolanic material; preferably selected from the group consisting of fly ash, silica fume, metakaolin, slag, and rice husk ash.; more preferably is silica fume.

According to one embodiment, the radiopacifier is selected from zirconium oxide, bismuth oxide, cerium oxide, barium sulphate, calcium tungstate, titanate dioxide, ytterbium oxide and mixtures thereof. In a specific embodiment, the radiopacifier is zirconium oxide. According to one embodiment, the setting accelerator is calcium carbonate, calcium oxide, calcium phosphate, sodium bicarbonate, calcium lactate, calcium chloride or mixtures thereof. According to one embodiment, the setting accelerator is calcium carbonate, calcium oxide or mixtures thereof. According to one embodiment, the setting accelerator is calcium chloride. According to one embodiment, the pigments may be iron oxides. According to one embodiment, the water-reducing agent is selected from glenium, polynaphtalene sulfonate, modified polycarboxylate. According to one embodiment, the texturing agents may be for example selected from silica, povidone (also named polyvinylpyrrolidone), cellulose or derivatives thereof such as methylcellulose, hydroxypropylcellulose and hydroxyethylcellulose, polymers such as acrylamide/sodium acryloyldimethyltaurate copolymer isohexadecane and hydroxyethyl acrylate/sodium acryloyl dimethyl taurate copolymer, mineral fillers, fumed silica (hydrophilic and/or hydrophobic), xanthan gum, or mixtures thereof. According to one embodiment, the pH stabilizing agent is a mineral acid or an organic acid. According to one embodiment, the surfactant is a polysorbate.

According to one embodiment, the (hardened) composite material comprises at least one additive in an amount ranging from 0% to 60% in weight to the total weight of the composite material; preferably from 2% to 50%; more preferably from 2% to 35%. According to one embodiment, the (hardened) composite material comprises at least one additive in an amount ranging from 0% to 30% in weight to the total weight of the composite material; preferably from 1% to 25%; more preferably from 1% to 18%.

According to one embodiment, the (hardened) composite material comprises from 0 to 40% of radiopacifier in weight to the total weight of said (hardened) composite material; preferably from 2 to 35%, from 5 to 35%, preferably 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35%. According to one embodiment, the (hardened) composite material comprises from 0 to 20% of radiopacifier in weight to the total weight of said (hardened) composite material; preferably from 1 to 18%, from 2 to 18%.

### Dispersant phase

According to one embodiment, the dispersant phase is a solid dispersant phase. According to one embodiment, the dispersant phase, preferably the solid dispersant phase, comprises or consists of at least one product of the hydration of a calcium silicate compound; preferably selected from tricalcium silicate, dicalcium silicate, Portland cement, mineral trioxide aggregate (MTA) and any combinations thereof; more preferably the solid dispersant phase comprises or consists of at least one product of the hydration of a hydraulic cement comprising tricalcium silicate.

According to one embodiment, the product(s) of the hydration of a calcium silicate compound may be a calcium silicate hydrate (CSH), calcium oxide (CaO) and/or a calcium hydroxide (Ca(OH)₂). According to one embodiment, the solid dispersant phase comprises or consists of at least one product of the hydration of a calcium silicate compound selected from calcium silicate hydrate (CSH), calcium oxide (CaO), calcium hydroxide (Ca(OH)₂) and mixtures thereof.

According to one embodiment, the calcium silicate hydrate (CSH) results from the hydration of tricalcium silicate (C3S) and/or dicalcium silicate (C2S).

According to one embodiment, the solid dispersant phase comprises or consists of at least one calcium silicate hydrate (CSH); preferably a calcium silicate hydrate of the following formula (I):

mCaO.nSiO₂.pH₂O

in which n and m independently range from 1 to 3 and p ranges from 3 to 6; preferably m equals 3, n equals 2 and p equals 3.

According to one embodiment, the solid dispersant phase comprises or consists of dense calcium silicate hydrates (d-CSH) as defined above.

According to one embodiment, the solid dispersant phase comprises or consists of porous calcium silicate hydrates (p-CSH) as defined above.

According to one embodiment, the solid dispersant phase comprises pores with a pore size ranging from more than 0 nm to 1 µm, preferably from 2 nm to 1 µm, more preferably from 2 nm to 100 nm, more preferably from 2 nm to 50 nm, more preferably from 2 nm to 20 nm, in particular from 8 nm to 15 nm.

According to one embodiment, the dispersant phase further comprises a pozzolanic material, preferably selected from the group consisting of: fly ash, silica fume, metakaolin, slag and rice husk ash; more preferably silica fume.

### Dispersed calcium silicate particles

According to one embodiment, the dio granulometry of calcium silicate particles in the composite material ranges from more than 0 µm to 2 µm; preferably from 0.1 µm to 2 µm. According to one embodiment, the dio granulometry of calcium silicate particles in the composite material is 0.1, 0.2, 0.3, 0.4, 0.5, 0.6, 0.7, 0.8, 0.9 or 1 µm. According to one embodiment, the dio granulometry of non-hydrated calcium silicate particles in the composite material ranges from more than 0 µm to 2 µm; preferably from 0.1 µm to 2 µm. According to one embodiment, the dio granulometry of non-hydrated calcium silicate particles in the composite material is 0.1, 0.2, 0.3, 0.4, 0.5, 0.6, 0.7, 0.8, 0.9 or 1 µm. According to one embodiment, the dio granulometry ranges as defined above are determined by laser diffraction.

According to one embodiment, the dso granulometry of calcium silicate particles in the composite material ranges from more than 0 µm to 10 µm; preferably from 1 µm to 8 µm; more preferably from 3 µm to 8 µm or from 0.5 µm to 3 µm. According to one embodiment, the dso granulometry of calcium silicate particles in the composite material is 1, 2, 3, 4, 5, 6, 7, 8, 9 or 10 µm. According to one embodiment, the dso granulometry of non-hydrated calcium silicate particles in the composite material ranges from more than 0 µm to 10 µm; preferably from 1 µm to 8 µm; more preferably from 3 µm to 8 µm or from 0.5 µm to 3 µm. According to one embodiment, the dso granulometry of non-hydrated calcium silicate particles in the composite material is 1, 2, 3, 4, 5, 6, 7, 8, 9 or 10 µm. According to one embodiment, the dso granulometry ranges as defined above are determined by laser diffraction.

According to one embodiment, the d₉₀ granulometry of calcium silicate particles in the composite material ranges from more than 0 µm to 20 µm; preferably from 1 µm to 10 µm; more preferably from 1 µm to 7 µm. According to one embodiment, the d₉₀ granulometry of calcium silicate particles in the composite material is 1, 2, 3, 4, 5, 6, 7, 8, 9, 10, 11, 12, 13, 14, 15, 16, 17, 18, 19 or 20 µm. According to one embodiment, the d₉₀ granulometry of non-hydrated calcium silicate particles in the composite material ranges from more than 0 µm to 20 µm; preferably from 1 µm to 10 µm; more preferably from 1 µm to 7 µm. According to one embodiment, the d₉₀ granulometry of non-hydrated calcium silicate particles in the composite material is 1, 2, 3, 4, 5, 6, 7, 8, 9, 10, 11, 12, 13, 14, 15, 16, 17, 18, 19 or 20 µm. According to one embodiment, the dso granulometry ranges as defined above are determined by laser diffraction.

According to one embodiment, the amount of calcium silicate particles in the composite material ranges from 5% wt. to 65% wt., preferably 8% wt. to 60% wt., more preferentially 10% wt. to 35% wt., by the total weight of said (hardened) composite material. According to one embodiment, the amount of non-hydrated calcium silicate particles ranges from 5% wt. to 65% wt., preferably 8% wt. to 60% wt., more preferentially 10% wt. to 35% wt., by the total weight of said (hardened) composite material.

According to one embodiment, the amount of calcium silicate particles in the composite material is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 35 or 35% wt. by the total weight of said (hardened) composite material. According to one embodiment, the amount of non-hydrated calcium silicate particles in the composite material is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 35 or 35% wt. by the total weight of said (hardened) composite material.

### Intergranular area

According to one embodiment, the (hardened) composite material comprises one or more intergranular area(s), preferably located between and/or around the particles of the calcium silicate compound as defined above. According to one embodiment, the (hardened) composite material comprises one or more intergranular area(s) located between and/or around non-hydrated calcium silicate particles dispersed in the dispersant phase as defined above.

According to one embodiment, the intergranular area(s) embed, partially or totally, one or more calcium silicate particles as defined above. According to one embodiment, the intergranular area(s) embed, partially or totally, one or more non-hydrated calcium silicate particles as defined above.

According to one embodiment, the intergranular area comprises or consists of calcium carbonate (CaCO₃), preferably under the form of particles and/or aggregates. According to one embodiment, the intergranular area comprises or consists of insoluble calcium carbonate (CaCO₃), preferably under the form of particles and/or aggregates. According to one embodiment, the intergranular area further comprises at least one product of the hydration of a calcium silicate compound as defined above; preferably at least one product of the hydration of a calcium silicate compound selected from tricalcium silicate, dicalcium silicate and mixtures thereof; more preferably at least one product of the hydration of tricalcium silicate. According to one embodiment, the intergranular area further comprises one or more calcium silicate hydrates (CSH).

According to one embodiment, the intergranular area comprises or consists of at least one calcium silicate hydrate (CSH) and calcium carbonate (CaCO₃), preferably under the form of particles and/or aggregates. According to one embodiment, the intergranular area comprises or consists of at least one calcium silicate hydrate (CSH) and insoluble calcium carbonate (CaCO₃), preferably under the form of particles and/or aggregates.

According to one embodiment, the calcium silicate hydrate (CSH) in the intergranular area is porous calcium silicate hydrate (p-CSH) as defined above. According to one embodiment, the calcium silicate hydrate (CSH) in the intergranular area is dense calcium silicate hydrate (d-CSH) as defined above. According to one embodiment, the intergranular area comprises or consists of porous calcium silicate hydrate(s) (p-CSH) and calcium carbonate (CaCO₃). According to one embodiment, the intergranular area comprises or consists of dense calcium silicate hydrate(s) (d-CSH) and calcium carbonate (CaCO₃).

According to one embodiment, the intergranular area is porous. According to one embodiment, the intergranular area comprises mesopores, micropores and/or macropores. According to one embodiment, the pore size is determined by mercury intrusion porosimetry (MIP). Alternatively, the pore size may be determined by Transmission Electron Microscopy (TEM).

The porosity of the intergranular area may vary in a wide range. In some embodiments the porosity of the intergranular area is quite low. A quite low porosity of the intergranular area contributes to reinforcing the mechanical properties of the composite material.

According to one embodiment, the (insoluble) calcium carbonate particles in the composite material of the invention have a dio granulometry ranging from 1 nm to 500 nm; preferably from 1 nm to 400 nm, from 1 nm to 300 nm, from 1 nm to 200 nm, from 1 nm to 100 nm, from 1 nm to 90 nm, from 1 nm to 80 nm, from 1 nm to 70 nm, from 1 nm to 60 nm, or from 1 nm to 50 nm. According to one embodiment, the (insoluble) calcium carbonate particles have a dio granulometry ranging from 400 nm to 500 nm. According to one embodiment, the (insoluble) calcium carbonate particles have a dio granulometry of 440 nm.

According to one embodiment, the (insoluble) calcium carbonate particles in the composite material of the invention have a dso granulometry ranging from 1 nm to 1500 nm; preferably from 1 nm to 1400 nm, from 1 nm to 1300 nm, from 1 nm to 1200 nm, from 1 nm to 1100 nm, from 1 nm to 1000 nm, from 1 nm to 900 nm, from 1 nm to 800 nm, from 1 nm to 700 nm, from 1 nm to 600 nm, from 1 nm to 500 nm, from 1 nm to 400 nm, from 1 nm to 300 nm, from 1 nm to 200 nm, from 1 nm to 100 nm, from 1 nm to 50 nm, or from 1 nm to 10 nm. According to one embodiment, the (insoluble) calcium carbonate particles have a dso granulometry ranging from 1000 nm to 1200 nm. According to one embodiment, the (insoluble) calcium carbonate particles have a dso granulometry of 1100 nm.

According to one embodiment, the (insoluble) calcium carbonate particles in the composite material of the invention have a d₉₀ granulometry ranging from 1 nm to 5 000 nm; preferably from 1 nm to 4000 nm, from 1 nm to 3000 nm, from 1 nm to 2000 nm, from 1 nm to 1000 nm, from 1 nm to 900 nm, from 1 nm to 800 nm, from 1 nm to 700 nm, from 1 nm to 600 nm, from 1 nm to 500 nm, from 1 nm to 400 nm, from 1 nm to 300 nm, from 1 nm to 200 nm, from 1 nm to 100 nm, from 1 nm to 50 nm, or from 1 nm to 10 nm. According to one embodiment, the (insoluble) calcium carbonate particles have a d₉₀ granulometry of 3000 nm to 3600 nm. According to one embodiment, the (insoluble) calcium carbonate particles have a d₉₀ granulometry of 3550 nm.

According to one embodiment, the amount of the (insoluble) calcium carbonate particles in the composite material of the invention ranges from more than 0% wt. to 20% wt., preferably from 1% wt. to 15% wt., more preferably from 1% wt. to 7% wt. by the total weight of said composite material. According to one embodiment, the amount of the (insoluble) calcium carbonate particles in the composite material of the invention is 1, 2, 3, 4, 5, 6, 7, 8, 9, 10, 11, 12, 13, 14, 15, 16, 17, 18, 19 or 20% wt., by the total weight of said composite material.

According to one embodiment, the intergranular area further comprises a pozzolanic material, preferably selected from the group consisting of: fly ash, silica fume, metakaolin, slag, and rice husk ash; more preferably silica fume.

According to one embodiment, the amount of the pozzolanic material in the composite material of the invention ranges from more than 0% wt. to 20% wt., preferably from 1% wt. to 15% wt., more preferably from 1% wt. to 7% wt. by the total weight of said composite material. According to one embodiment, the amount of the pozzolanic material in the composite material of the invention is 1, 2, 3, 4, 5, 6, 7, 8, 9, 10, 11, 12, 13, 14, 15, 16, 17, 18, 19 or 20% wt., by the total weight of said composite material.

### Specific composite material

According to one embodiment, the (hardened) composite material comprises or consists of:
- a dispersant phase, preferably a solid dispersant phase, comprising or consisting of calcium silicate hydrates (CSH);
- calcium silicate particles, preferably tricalcium silicate particles, dispersed in the dispersant phase; and
- one or more intergranular area(s) comprising or consisting of insoluble calcium carbonate particles; said insoluble calcium carbonate particles having a dso granulometry ranging from 1 nm to 1500 nm; preferably from 1 nm to 1000 nm.

According to one embodiment, the (hardened) composite material comprises or consists of:
- a dispersant phase, preferably a solid dispersant phase, comprising or consisting of calcium silicate hydrates (CSH);
- calcium silicate particles, preferably tricalcium silicate particles, dispersed in the dispersant phase; and
- one or more intergranular area(s) comprising or consisting of at least one pozzolanic material and insoluble calcium carbonate particles;
said insoluble calcium carbonate particles having a dso granulometry ranging from 1 nm to 1500 nm; preferably from 1 nm to 1000 nm.

According to one embodiment, the (hardened) composite material comprises or consists of:
- a dispersant phase, preferably a solid dispersant phase, comprising or consisting of calcium silicate hydrates (CSH);
- from 5% wt. to 65% wt. of calcium silicate particles, preferably tricalcium silicate particles, dispersed in the dispersant phase, by the total weight of the composite material; and
- one or more intergranular area(s) comprising or consisting of insoluble calcium carbonate particles;
said insoluble calcium carbonate particles having a dso granulometry ranging from 1 nm to 1500 nm; preferably from 1 nm to 1000 nm;

According to one embodiment, the amount of the insoluble calcium carbonate particles ranges from 1% wt. to 20% wt. by the total weight of said composite material.

According to one embodiment, the (hardened) composite material comprises or consists of:
- a dispersant phase, preferably a solid dispersant phase, comprising or consisting of calcium silicate hydrates (CSH);
- from 5% wt. to 65% wt. of calcium silicate particles, preferably tricalcium silicate particles, dispersed in the dispersant phase, by the total weight of the composite material; and
- one or more intergranular area(s) comprising or consisting of at least one pozzolanic material and insoluble calcium carbonate particles;
said insoluble calcium carbonate particles having a dso granulometry ranging from 1 nm to 1500 nm; preferably from 1 nm to 1000 nm; optionally,
the amount of the at least one pozzolanic material ranges from 1% wt. to 15% wt. by the total weight of said composite material; and
the amount of the insoluble calcium carbonate particles ranges from 1% wt. to 5% wt. by the total weight of said composite material.

According to one embodiment, the (hardened) composite material comprises or consists of:
- a solid dispersant phase comprising or consisting of calcium silicate hydrates (CSH);
- from 5% wt. to 65% wt. of non-hydrated tricalcium silicate particles dispersed in the dispersant phase, by the total weight of the composite material; and
- one or more intergranular area(s) comprising or consisting of insoluble calcium carbonate particles;
said insoluble calcium carbonate particles having a dso granulometry ranging from 1 nm to 1500 nm; preferably from 1 nm to 1000 nm;
optionally, the amount of the insoluble calcium carbonate particles ranges from 1% wt. to 20% wt. by the total weight of said composite material.

According to one embodiment, the (hardened) composite material comprises or consists of:
- from 5% to 65%, preferably from 8% to 60%, more preferentially from 10% to 35% by weight with respect to the total weight of the (hardened) composite material, of calcium silicate particles, said calcium silicate particles preferably being chosen from the group comprising tricalcium silicate, dicalcium silicate, Portland cement, mineral trioxide aggregates (MTA) and/or mixtures thereof,
- from 1% to 20%, preferably from 2% to 15% by weight with respect to the total weight of the (hardened) composite material, of calcium carbonate, preferably insoluble calcium carbonate particles; and
- from more than 0% to 50%, preferably from 1% to 40% by weight with respect to the total weight of the (hardened) composite material, of mCaO.nSiO₂.pH₂O (CSH) in which m and n, each independently, vary from 1 to 3 and p varies from 3 to 6.

According to one embodiment, the (hardened) composite material comprises or consists of:
- from more than 0% to 50%, preferably from 1% to 40% by weight with respect to the total weight of the (hardened) composite material, of a dispersant phase comprising or consisting of mCaO.nSiO₂.pH₂O (CSH) in which m and n, each independently, vary from 1 to 3 and p varies from 3 to 6;
- from 5% to 65%, preferably from 8% to 60%, more preferentially from 10% to 35% by weight with respect to the total weight of the (hardened) composite material, of calcium silicate particles, said calcium silicate particles preferably being chosen from the group comprising tricalcium silicate, dicalcium silicate, Portland cement, mineral trioxide aggregates (MTA) and/or mixtures thereof; said calcium silicate particles being dispersed in the dispersant phase; and
- from 1% to 20%, preferably from 2% to 15% by weight with respect to the total weight of the (hardened) composite material, of calcium carbonate, preferably insoluble calcium carbonate particles; said insoluble calcium carbonate particles be located in one or more intergranular area(s) located between and/or around the calcium silicate particles.

According to one embodiment, the (hardened) composite material has a compressive strength measured 24h after mixing the powder phase (or the hydraulic cement) and the aqueous phase, ranging from more than 0 MPa to 400 MPa, preferably from 10 MPa to 300 MPa, more preferably from 50 MPa to 250 MPa.

### Process for manufacturing said composite

The invention also relates to a method for manufacturing a (hardened) composite material, comprising at least one mixing step of a powder phase with an aqueous phase. According to one embodiment, the method for manufacturing a (hardened) composite material, comprises at least one mixing step of a powder phase comprising or consisting of a hydraulic cement, preferably a hydraulic calcium silicate cement, with an aqueous phase. According to one embodiment, the aqueous phase is an aqueous liquid or an aqueous solution.

According to one embodiment, the method for manufacturing a (hardened) composite material, comprises at least one mixing step of a powder phase comprising calcium silicate particles and calcium carbonate particles, with an aqueous phase. According to one embodiment, the method for manufacturing a (hardened) composite material, comprises at least one mixing step of a powder phase comprising calcium silicate particles, at least one pozzolanic material and calcium carbonate particles, with an aqueous phase. According to one embodiment, the method for manufacturing a (hardened) composite material, comprises at least one mixing step of a powder phase comprising calcium silicate particles, at least one pozzolanic material selected from the group consisting of: fly ash, silica fume, metakaolin, slag, and rice husk ash; and calcium carbonate particles, with an aqueous phase.

According to one embodiment, the powder phase is anhydrous. According to one embodiment, the powder phase is a hydraulic cement. According to one embodiment, the powder phase comprises or consists of a calcium silicate compound, preferably under the form of particles. According to one embodiment, the calcium silicate compound is selected from tricalcium silicate, dicalcium silicate, Portland cement and/or mineral trioxide aggregates (MTA). According to one embodiment, the powder phase is an anhydrous calcium silicate cement powder phase.

Without wishing to be bound by any theory, it seems that the specific features of the process of the invention afford obtaining a porous intergranular area which porosity is not too high, thus reinforcing the mechanical properties of the composite material.

### Calcium silicate granulometry

According to one embodiment, the dio granulometry of calcium silicate particles in the mixture ranges from more than 0 µm to 2 µm; preferably from 0.1 µm to 2 µm. According to one embodiment, the dio granulometry of calcium silicate particles in the mixture is 0.1, 0.2, 0.3, 0.4, 0.5, 0.6, 0.7, 0.8, 0.9 or 1 µm. According to one embodiment, the dio granulometry of non-hydrated calcium silicate particles in the mixture ranges from more than 0 µm to 2 µm; preferably from 0.1 µm to 2 µm. According to one embodiment, the dio granulometry of non-hydrated calcium silicate particles in the mixture is 0.1, 0.2, 0.3, 0.4, 0.5, 0.6, 0.7, 0.8, 0.9 or 1 µm. According to one embodiment, the dio granulometry ranges as defined above are determined by laser diffraction.

According to one embodiment, the dso granulometry of calcium silicate particles in the mixture ranges from more than 0 µm to 10 µm; preferably from 1 µm to 8 µm; more preferably from 3 µm to 8 µm or from 0.5 µm to 3 µm. According to one embodiment, the dso granulometry of calcium silicate particles in the mixture is 1, 2, 3, 4, 5, 6, 7, 8, 9 or 10 µm. According to one embodiment, the dso granulometry of non-hydrated calcium silicate particles in the mixture ranges from more than 0 µm to 10 µm; preferably from 1 µm to 8 µm; more preferably from 3 µm to 8 µm or from 0.5 µm to 3 µm. According to one embodiment, the dso granulometry of non-hydrated calcium silicate particles in the mixture is 1, 2, 3, 4, 5, 6, 7, 8, 9 or 10 µm. According to one embodiment, the dso granulometry ranges as defined above are determined by laser diffraction.

According to one embodiment, the d₉₀ granulometry of calcium silicate particles in the mixture ranges from more than 0 µm to 20 µm; preferably from 1 µm to 10 µm; more preferably from 1 µm to 7 µm. According to one embodiment, the d₉₀ granulometry of calcium silicate particles in the mixture is 1, 2, 3, 4, 5, 6, 7, 8, 9, 10, 11, 12, 13, 14, 15, 16, 17, 18, 19 or 20 µm. According to one embodiment, the d₉₀ granulometry of non-hydrated calcium silicate particles in the mixture ranges from more than 0 µm to 20 µm; preferably from 1 µm to 10 µm; more preferably from 1 µm to 7 µm. According to one embodiment, the d₉₀ granulometry of non-hydrated calcium silicate particles in the mixture is 1, 2, 3, 4, 5, 6, 7, 8, 9, 10, 11, 12, 13, 14, 15, 16, 17, 18, 19 or 20 µm. According to one embodiment, the dso granulometry in the mixture ranges as defined above are determined by laser diffraction.

### Calcium carbonate granulometry

According to one embodiment, calcium carbonate particles in the mixture have a dio granulometry ranging from 1 nm to 1000 nm; preferably from 1 nm to 900 nm, from 1 nm to 800 nm, from 1 nm to 700 nm, from 1 nm to 600 nm, from 1 nm to 500 nm, from 1 nm to 400 nm, from 1 nm to 300 nm, from 1 nm to 200 nm, or from 1 nm to 100 nm. According to one embodiment, calcium carbonate particles in the mixture have a dio granulometry ranging from 500 nm to 800 nm. According to one embodiment, calcium carbonate particles in the mixture have a dio granulometry of 600 nm.

According to one embodiment, calcium carbonate particles in the mixture have a dso granulometry ranging from 1 nm to 5 000 nm; preferably from 1 nm to 4000 nm, from 1 nm to 3000 nm, from 1 nm to 2000 nm, from 1 nm to 1000 nm, from 1 nm to 900 nm, from 1 nm to 800 nm, from 1 nm to 700 nm, from 1 nm to 600 nm, from 1 nm to 500 nm, from 1 nm to 400 nm, from 1 nm to 300 nm, from 1 nm to 200 nm, or from 1 nm to 100 nm. According to one embodiment, calcium carbonate particles in the mixture have a dso granulometry ranging from 2500 nm to 3000 nm. According to one embodiment, calcium carbonate particles in the mixture have a dso granulometry of 2760 nm.

According to one embodiment, calcium carbonate particles in the mixture have a d₉₀ granulometry ranging from 1 nm to 30 000 nm; preferably from 1 nm to 29 000 nm, from 1 nm to 27 000 nm, from 1 nm to 26 000 nm, from 1 nm to 25 000 nm, from 1 nm to 24 000 nm, from 1 nm to 23 000 nm, from 1 nm to 22 000 nm, from 1 nm to 21 000 nm, from 1 nm to 20 000 nm, from 1 nm to 19 000 nm, from 1 nm to 18 000 nm, from 1 nm to 17 000 nm, from 1 nm to 16 000 nm, from 1 nm to 15 000 nm, from 1 nm to 14 000 nm, from 1 nm to 13 000 nm, from 1 nm to 12 000 nm, from 1 nm to 11 000 nm, from 1 nm to 10 000 nm, from 1 nm to 9 000 nm, from 1 nm to 8 000 nm, from 1 nm to 7 000 nm, from 1 nm to 6 000 nm, from 1 nm to 5 000 nm, from 1 nm to 4000 nm, from 1 nm to 3000 nm, from 1 nm to 2000 nm, from 1 nm to 1000 nm, from 1 nm to 900 nm, from 1 nm to 800 nm, from 1 nm to 700 nm, from 1 nm to 600 nm, from 1 nm to 500 nm, from 1 nm to 400 nm, from 1 nm to 300 nm, from 1 nm to 200 nm or from 1 nm to 100 nm. According to one embodiment, calcium carbonate particles in the mixture have a d₉₀ granulometry ranging from 25000 nm to 30000 nm. According to one embodiment, calcium carbonate particles in the mixture have a d₉₀ granulometry of 27400 nm.

### Calcium silicate amount

According to one embodiment, the powder phase comprises calcium silicate particles in an amount ranging from 10% to 100% by weight of the total weight of the powder phase, preferably from 10% to 98%, preferably from 15% to 60%, more preferably from 20 to 55%.

According to one embodiment, the powder phase, the aqueous phase and/or the mixture further comprises at least one additive; preferably selected from setting accelerator, radiopacifiers, pigments, pH stabilizing agents, fillers, texturing/thickening agents, water-reducing agents and mixtures thereof. According to one embodiment, the filler is a pozzolanic material; preferably selected from the group consisting of fly ash, silica fume, metakaolin, slag, and rice husk ash.; more preferably is silica fume.

According to one embodiment, the radiopacifier is selected from zirconium oxide, bismuth oxide, cerium oxide, barium sulphate, calcium tungstate, titanate dioxide, ytterbium oxide and mixtures thereof. In a specific embodiment, the radiopacifier is zirconium oxide. According to one embodiment, the setting accelerator is calcium carbonate, calcium oxide, calcium phosphate, sodium bicarbonate, calcium lactate, calcium chloride or mixtures thereof. According to one embodiment, the setting accelerator is calcium carbonate, calcium oxide or mixtures thereof. According to one embodiment, the setting accelerator is calcium chloride. According to one embodiment, the pigments may be iron oxides. According to one embodiment, the water-reducing agent is selected from glenium, polynaphtalene sulfonate, modified polycarboxylate. According to one embodiment, the texturing agents may be for example selected from silica, povidone (also named polyvinylpyrrolidone), cellulose or derivatives thereof such as methylcellulose, hydroxypropylcellulose and hydroxyethylcellulose, polymers such as acrylamide/sodium acryloyldimethyltaurate copolymer isohexadecane and hydroxyethyl acrylate/sodium acryloyl dimethyl taurate copolymer, mineral fillers, fumed silica (hydrophilic and/or hydrophobic), xanthan gum, or mixtures thereof. According to one embodiment, the pH stabilizing agent is a mineral acid or an organic acid. According to one embodiment, the surfactant is a polysorbate.

According to one embodiment, the powder phase comprises or consists of:
- 85% of at least one calcium silicate compound, preferably selected from tricalcium silicate, dicalcium silicate and mixtures thereof; and
- 15% of calcium carbonate; in weight to the total weight of the powder phase.

According to one embodiment, the powder phase comprises or consists of:
- 50% of at least one calcium silicate compound, preferably selected from tricalcium silicate, dicalcium silicate and mixtures thereof; and
- 50% of calcium carbonate; in weight to the total weight of the powder phase.

According to one embodiment, the powder phase, the aqueous phase and/or the mixture comprise(s) at least one additive in an amount ranging from 0% to 60% in weight to the total weight of the mixture; preferably from 2% to 50%; more preferably from 2% to 35%. According to one embodiment, the powder phase, the aqueous phase and/or the mixture comprise(s) at least one additive in an amount ranging from 0% to 30% in weight to the total weight of the mixture; preferably from 1% to 25%; more preferably from 1% to 18%.

According to one embodiment, the powder phase, the aqueous phase and/or the mixture comprise(s) from 0 to 40% of radiopacifier in weight to the total weight of said mixture; preferably from 2 to 35%, from 5 to 35%, preferably 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35%. According to one embodiment, the powder phase, the aqueous phase and/or the mixture comprise(s) from 0 to 20% of radiopacifier in weight to the total weight of said mixture; preferably from 1 to 18%, from 2 to 18%.

According to one embodiment, the mass ratio of the powder phase to the aqueous phase ranges from 2 to 4.5. According to one embodiment, the mass ratio of the powder phase to the aqueous phase is 2,1; 2,2; 2,3; 2,4; 2,5; 2,6; 2,7; 2,8; 2,9; 3.0; 3.1; 3.2; 3,3; 3,4; 3,5; 3,6; 3,7; 3,8; 3,9; 4.0; 4,1; 4,2; 4,3; 4,4 or 4,5.

According to one embodiment, the powder phase comprises:
- calcium silicate particles;
- more than 0% to 15% of at least one pozzolanic material; preferably selected from the group consisting of fly ash, silica fume, metakaolin, slag, and rice husk ash; more preferably silica fume; and
- more than 0% to 15% of calcium carbonate; in weight to the total weight of the powder phase.

According to one embodiment, the powder phase comprises from:
- calcium silicate particles;
- more than 0% to 15% of at least one pozzolanic material; preferably selected from the group consisting of fly ash, silica fume, metakaolin, slag, and rice husk ash; more preferably silica fume; and
- more than 0% to 15% of calcium carbonate; in weight to the total weight of the mixture.

As mentioned above, dental restoration material of the invention may result from the mixture of the calcium silicate powder phase described above with an aqueous liquid phase.

According to one embodiment, the aqueous liquid phase comprises water, preferably purified water.

According to one embodiment, the aqueous liquid phase consists in water. In another embodiment, the aqueous liquid phase is an aqueous solution

According to one embodiment, the aqueous liquid phase comprises from 10 to 100% of water, in weight to the total weight of said aqueous liquid phase, preferably from 20% to 90%, preferably from 30% to 90%, preferably from 35% to 85%. According to one embodiment, the liquid phase comprises from 50% to 90% of water in weight to the total weight of said liquid phase, preferably from 60% to 90%, more preferably from 60% to 85%, more preferably from 65% to 85 %.

According to one embodiment, the aqueous phase is an aqueous liquid phase and comprises at least one additive, wherein the additive is preferably selected from setting accelerators and water reducing agents. According to one embodiment, the aqueous liquid phase comprises one or more additives selected from setting accelerators (such as calcium chloride), water reducing agents (such as modified polycarboxylate, glenium, polynaphthalene sulfonate or mixtures thereof) and mixtures thereof.

According to one embodiment, the aqueous liquid phase comprises at least one additive in an amount ranging from 0% to 40% in weight to the total weight of the liquid phase; preferably from 10% to 35%; more preferably from 15% to 35%.

According to one embodiment, the mixing step is implemented by a vibration mixer.

According to one embodiment, the method for manufacturing a composite material as defined above, comprises at least one mixing step by vibration of a powder phase comprising calcium silicate particles and calcium carbonate particles, with an aqueous phase; preferably in a mass ratio of the powder phase to the aqueous phase ranging from 2 to 4.5.

According to one embodiment, the mixing step is implemented with a vibration frequency ranging from 1 rpm to 10 000 rpm; preferably ranging from 1000 rpm to 6 000 rpm; more preferably ranging from 3 000 rpm to 5 000 rpm. According to one embodiment, the mixing step is implemented with a vibration frequency of about 1, 100, 200, 300, 400, 500, 600, 700, 800, 900, 1 000, 2 000, 3 000, 4 000, 5 000, 6 000, 7 000, 8 000, 9 000 or 10 000 rpm.

According to one embodiment, the mixing step by vibration is implemented during a vibration time ranging from 1 s to 3600 s; preferably from 1 s to 60 s; more preferably during 30 s. According to one embodiment, the mixing step by vibration is implemented during a vibration time of 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 s.

### Uses and methods for treating a subject

Another object of the present invention is the use of the composite material as defined above. According to one embodiment, the (hardened) composite material of the invention is useful as reinforcing material in the non-therapeutical cement field. According to one embodiment, the non-therapeutical cement field is the constructive field. According to one embodiment, the composite material of the invention is for use in the medical field, preferably in the dental or the orthopedic field, as a restorative and/or filling material.

According to one embodiment, the composite materials of the invention may also be used in orthopedics, in bone restoration, in craniofacial and/or maxillofacial surgery.

Another object of the present invention relates to a method for treating the crown of a tooth, for example enamel restoration, permanent dentin restoration, deep or large carious lesions restoration, deep cervical or radicular lesions restoration, pulp capping or pulpotomy; and/or the root of a tooth, such as for example root and furcation perforations, internal/external resorptions, apexification or retrograde surgical filling; in a subject in need thereof, comprising the use of a composite material of the invention as defined above.

According to one embodiment, the composite materials of the invention may be used in treating a bone and/or dental disorder or disease in a subject in need thereof. According to one embodiment, the present invention refers to the use of the composite materials of the invention for treating a bone and/or dental disorder or disease in a subject in need thereof. According to one embodiment, the present invention refers to a method for treating a bone and/or dental disorder or disease in a subject in need thereof by using the composite materials of the invention.

### EXAMPLES

The present invention is further illustrated by the following examples.

### Example 1: Effects of mixing parameters on the granulometry of the components of the powder phase (hydraulic calcium silicate cement)

The Applicant has studied the effect of the mixing parameters used in the process for manufacturing a hardened cementitious material from a hydraulic calcium silicate cement, on the final granulometry of the components of said cement.

For this goal, the Applicant has studied:
- the granulometry of a powder phase A consisting of calcium silicate particles alone,
- the granulometry of a powder phase B consisting of calcium carbonate particles alone, and
- the granulometry of a powder phase C consisting of 15% of tricalcium silicate particles and 85% of calcium carbonate particles in weight to the total weight of the powder phase.

Each powder phase has been stirred under vibration with a vibration frequency ranging from 3000 rpm to 3500 rpm, during 30s.

Then, the d10, d50 and d90 granulometries for each powder phase have been determined by laser diffraction and compared to their initial d10, d50 and d90 granulometries.

The results are shown in the following Table.

| **Powder phase** | **d₁₀** | | **d₅₀** | | **d₉₀** | |
|---|---|---|---|---|---|---|
| | **(µM)** | | **(µM)** | | **(µM)** | |
| | Before vibration | After vibration | Before vibration | After vibration | Before vibration | After vibration |
| A | 1.59 | 0.81 | 3.86 | 3.56 | 7.87 | 7.1 |
| B | 0.60 | 0.44 | 2.76 | 1.1 | 27.4 | 3.55 |
| C* | 0.49 | 0.45 | 2.12 | 1.31 | 19.5 | 5.12 |

| | | | | | | |
|---|---|---|---|---|---|---|
| * *determination of the granulometry based on the calcium carbonate refractory index.* | | | | | | |

The results show that the mixing parameters used in the process for manufacturing the composite material from a hydraulic cement lead to a self-griding of calcium silicate particles and calcium carbonate particles. Especially, the d₁₀, d₅₀ and d₉₀ granulometries are more reduced for calcium carbonate particles than for calcium silicate particles.

The self-griding phenomenon is also observed when the calcium silicate and the calcium carbonate particles are mixed together.

### Example 2: Process for manufacturing a hardened composite material of the invention

The Applicant has manufactured hardened cementitious materials from a hydraulic calcium silicate cement which is powder phase C as defined in example 1.

For this aim, the Applicant has mixed the powder phase C with an aqueous liquid phase in a mass ratio of the powder phase to the aqueous phase ranging from 2 to 4.5, using a vibration mixer with a vibration frequency ranging from 3000 rpm to 3 500 rpm during 30s.

Surprisingly, even with a low amount (15%) of tricalcium silicate linker, an efficient setting and a short setting time (around 30 minutes) were obtained with powder phase C.

The obtained material was observed by microscopy and featured a composite structure comprising:
- a solid dispersant phase of calcium silicate hydrates (CSH);
- tricalcium silicate particles dispersed in the solid dispersant phase; and
- porous intergranular areas located between and around said tricalcium silicate particles; said porous intergranular areas comprising insoluble calcium carbonate particles having a d₁₀, d₅₀ and d₉₀ granulometry reduced compared to the initial d₁₀, d₅₀ and d₉₀ granulometry of said calcium carbonate particles.

## Claims

1. A composite material comprising:
- a solid dispersant phase comprising or consisting of calcium silicate hydrates (CSH);
- calcium silicate particles dispersed in the solid dispersant phase;
- a porous intergranular area located between said calcium silicate particles; said porous intergranular area comprising insoluble calcium carbonate particles having a dso granulometry ranging from 1 nm to 1500 nm; preferably from 1 nm to 1000 nm.

2. The composite material according to claim **1,** wherein the calcium silicate particles are selected from tricalcium silicate (C3S), dicalcium silicate (C2S) and any combinations thereof; preferably the calcium silicate particles are tricalcium silicate particles.

3. The composite material according to claim **1** or claim **2,** wherein the calcium silicate hydrates are compounds of formula (I):
mCaO.nSiO₂.pH₂O
in which n and m independently range from 1 to 3 and p ranges from 3 to 6; preferably m equals 3, n equals 2 and p equals 3.

4. The composite material according to any one of claims **1** to **3,** wherein the composite material further comprises at least one additive, preferably selected from set accelerators, radiopacifiers, pigments, pH stabilizing agents, fillers, texturing/thickening agents, water-reducing agents and mixtures thereof.

5. The composite material according to claim **4,** wherein the radiopacifier is selected from zirconium oxide, bismuth oxide, cerium oxide, barium sulphate, calcium tungstate, titanate dioxide, ytterbium oxide and mixtures thereof; preferably the radiopacifier is zirconium oxide.

6. The composite material according to any one of claims **1** to **5,** wherein the porous intergranular area further comprises calcium hydroxide (Ca(OH)₂).

7. The composite material according to any one of claims **1** to **6,** wherein the diameter of the pores of the porous intergranular area ranges from more than 0 nm to 50 nm.

8. The composite material according to any one of claims **1** to 7, wherein the dso granulometry of calcium silicate particles ranges from 1 µm to 10 µm; preferably from 1 µm to 8 µm.

9. The composite material according to any one of claims **1** to **8,** wherein the amount of calcium silicate particles ranges from 5 % wt. to 65 % wt. by the total weight of said composite material.

10. The composite material according to any one of claims **1** to **9,** wherein the porous intergranular area further comprises calcium silicate hydrates (CSH).

11. A method for manufacturing a composite material according to any one of claims **1** to **10,** comprising at least one mixing step by vibration of a powder phase comprising calcium silicate particles and calcium carbonate particles, with an aqueous phase; in a mass ratio of the powder phase to the aqueous phase ranging from 2 to 4.5.

12. The method according to claim **11,** wherein the mixing step is implemented with a vibration frequency ranging from 1 rpm to 15 000 rpm; preferably ranging from 1000 rpm to 6 000 rpm; more preferably ranging from 3 000 rpm to 5 000 rpm.

13. The method according to claim **11** or claim **12,** wherein the mixing step by vibration is implemented during a vibration time ranging from 1 s to 3600 s; preferably from 1 s to 60 s; more preferably during 30 s.

14. Use of a composite material according to any one of claims **1** to **10,** as reinforcing material in the non-therapeutical cement field.

15. The composite material according to any one of claims **1** to **10,** for use in the medical field, preferably in the dental or the orthopedic field, as a restorative and/or filling material.
